# EUROPÄISCHE PATENTSCHRIFT

(11) **EP 2 550 963 B1**
(45) Veröffentlichungstag und Bekanntmachung des Hinweises auf die Patenterteilung: **12.10.2016**
(21) Anmeldenummer: 11175252.3
(22) Anmeldetag: 25.07.2011
(51) Int. Cl.: A61K 31/222, C07C 259/18, A61P 33/02, A61P 33/06

(54) **Pentamidin-Amidoximsäureesters als Prodrugs und ihre Verwendung als Arzneimittel**
Amidoxime carboxylic acid esters of pentamidine as prodrugs and their use as medicament
Esters d'acide carboxylique d'amidoxime de la pentamidine en tant que prodrogues et leur utilisation comme médicament

(43) Veröffentlichungstag der Anmeldung: 30.01.2013
(73) Patentinhaber: Dritte Patentportfolio Beteiligungsgesellschaft mbH & Co. KG, 12529 Schönefeld (DE)
(72) Erfinder: Clement, Bernd, 24106 Kiel (DE); Kotthaus, Joscha, 24116 Kiel (DE); Kotthaus, Jürke, 24118 Kiel (DE); Schade, Dennis, La Jolla, CA California 92037 (US)
(74) Vertreter: Zech, Stefan Markus

(56) Entgegenhaltungen:
- US-A- 5 786 383
- US-A1- 2011 028 756
- JOSCHA KOTTHAUS ET AL: "Synthesis and biological evaluation of l-valine-amidoximeesters as double prodrugs of amidines", BIOORGANIC & MEDICINAL CHEMISTRY, Bd. 19, Nr. 6, 1. März 2011 (2011-03-01), Seiten 1907-1914, XP55007627, ISSN: 0968-0896, DOI: 10.1016/j.bmc.2011.01.066

## Beschreibung

Die vorliegende Erfindung betrifft Prodrug-Derivate des Pentamidins, ihre Verwendung zur Behandlung und/oder Prophylaxe von Krankheiten, insbesondere von Tumor- und Krebserkrankungen, sowie der Leishmaniose, Trypanosomiasis, der Pneumocystis carinii Pneumonie (PcP), sowie der Malaria. Pentamidin ist eine antiparasitär und antimikrobiell wirksame Verbindung, deren Verwendung in der Behandlung von Trypanosomiasis, Leishmaniose sowie Pneumocystis carinii Pneumonie (PcP) etabliert ist. Aufgrund der zwei stark basischen Amidinfunktionen ist die Verbindung unter physiologischen Bedingungen geladen und wird nach oraler Applikation nicht vom Organismus aufgenommen. Aus diesem Grund muss die Verbindung parenteral, z.B. intramuskulär, intravenös oder inhalativ, verabreicht werden. Zu bedenken ist hierbei, dass die meisten Infektionen durch die zuvor genannten Pathogene in tropischen und subtropischen Ländern auftreten, in denen die medizinische Versorgung oft ungenügend ist. Aufwendige Applikationsformen, wie es die intravenöse und inhalative Applikation darstellen, erschweren somit gerade in diesen Ländern eine sichere Arzneimittel-Therapie. Aus diesem Grund ist die Entwicklung eines oral bioverfügbaren Pentamidin-Prodrugs von enormer Bedeutung, um die Behandlungsmöglichkeiten entscheidend zu verbessern. Ein weiterer negativer Aspekt ist die nicht vorhandene ZNS-Gängigkeit des Pentamidins, was dazu führt, dass Pentamidin nur im Frühstadium der Trypanosomiasis (Afrikanische Schlafkrankheit) wirksam ist und nicht in der meningoenzephalitischen Phase, bei der die Erreger in das ZNS penetrieren.

Ein weiteres mögliches Einsatzgebiet des Pentamidins liegt in der Krebs-Therapie. Die hemmende Wirkung des Pentamidins auf die Endo-Exonuklease wurde in den vergangenen Jahren eingehend studiert.^{1,2} Erste klinische Studien zeigten bereits aussichtsreiche Ergebnisse bei der Behandlung von Brust- und Colon-Karzinomen.³ Auch hier ist der Einsatz eines oral verfügbaren Pentamidin-Prodrugs von großer Bedeutung.

Aus diesen Gründen wurden zahlreiche Versuche durchgeführt, um sowohl die orale Bioverfügbarkeit als auch die ZNS-Gängigkeit des Pentamidins zu verbessern. In vorangegangenen Arbeiten wurde das Pentamidin in das weniger basische Pentamidin-Diamidoxim überführt, was eine starke Erhöhung der Lipophilie zur Folge hat. Da Amidoxime bei physiologischen Bedingungen ungeladen vorliegen, ist die Absorption dieser Verbindungen aus dem Gastro-Intestinal-Trakt drastisch erhöht.⁴ Die ausgeprägte Reduktion der Amidoxime in die pharmakologisch aktiven Amidine konnte erstmalig im Jahr 1988 an der Modell-Verbindung Benzamidoxim gezeigt werden.⁵ Später wurde das Prinzip auf das Pentamidin übertragen, wodurch das Pentamidin-Monoamidoxim und Pentamidin-Diamidoxim **(3)** erhalten wurden. In Tierstudien zeigten beide Verbindungen eine geringe orale Bioverfügbarkeit und eine gute Aktivierbarkeit in die Wirkform Pentamidin.⁶ Inzwischen konnte das für die Reduktion verantwortliche Enzymsystem als ein bisher unbekanntes Molybdän-haltiges System identifiziert werden, welches mARC (mitochondrial Amidoxime Reducing Component) genannt wurde.^{7,8}

Um sowohl das pharmakokinetische Profil zur Verbesserung der Bioverfügbarkeit als auch die ZNS-Gängigkeit zu optimieren, wurden weitere Prodrugs entwickelt. Mit dem N,N-bis(acetoxy)pentamidin wurde eine Verbindung erhalten, die im Vergleich zu anderen Pentamidin-Prodrugs über eine deutlich erhöhte Lipophilie verfügt. Auch dieses Prodrug konnte in Tierstudien orale Bioverfügbarkeit an sowohl Ratten als auch an Schweinen zeigen. Nachteil des N,N-bis(acetoxy)pentamidin ist zum einen seine sehr geringe Wasserlöslichkeit, zum anderen war die ermittelte Bioverfügbarkeit sehr gering und es konnte keine ZNS-Gängigkeit nachgewiesen werden.⁹ Ähnliche Ansätze führten zur Entwicklung des N,N'-bis(methoxy)pentamidins, welches ähnlich dem N,N'-bis(acetoxy)pentamidin über eine sehr geringe Wasserlöslichkeit verfügt. Weitere Prodrug-Prinzipien, die auf das Pentamidin übertragen wurden sind die Hydroxylierung zum N,N'-bis(dihydroxy)pentamidin und die Konjugation mit Aminosäuren (insbesondere Valin) zum N,N'-bis(valoxy)pentamidin.¹⁰⁻¹² Zusammenfassend ist festzustellen, dass bis heute kein Pentamidin-Prodrug entwickelt werden konnte, das die erforderlichen Kriterien (gute orale Bioverfügbarkeit, ZNS-Gängigkeit und gute Löslichkeit) optimal erfüllt.

Vor diesem Hintergrund lag der vorliegenden Erfindung die Aufgabe zugrunde, Pentamidin-Prodrugs bereitzustellen, die gegenüber den bekannten Prodrugs von Pentamidin verbesserte Eigenschaften aufweisen.

Die genannte Aufgabe wird erfindungsgemäß gelöst durch eine Verbindung der Formel (I) 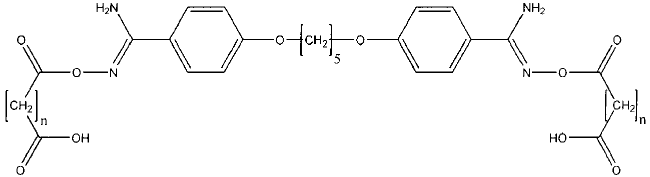 in welcher n für 2 steht.

Erfindungsgemäß steht n in Formel (I) für 2.

In einer weiteren bevorzugten Ausführungsform steht n in Formel (I) für 3. In einer bevorzugten Ausführungsform steht n in Formel (I) für 1, 3, 4, 5, 6, 7, 8, 9 oder 10.

Insbesondere das N;N'-Bis(succinyloxy)pentamidin **(1)** ist gegenüber den bisher beschriebenen Pentamidin-Prodrugs klar überlegen. Es wurde insbesondere eine erhebliche Verbesserung der Löslichkeit festgestellt, die einen sehr kritischen Parameter anderer Pentamidin-Prodrugs darstellt. Durch diese verbesserte Löslichkeit wird das pharmakokinetische Verhalten der Substanz positiv beeinflusst, da gute Lösungseigenschaften einen wichtigen Parameter bei der Absorption von Arzneistoffen darstellen.
Die vorliegende Erfindung betrifft darüber hinaus auch Salze, Solvate und Solvate der Salze der genannten Verbindungen der Formel (I). Ferner betrifft die vorliegende Erfindung die genannten Verbindungen der Formel (I) zur Behandlung und/oder Prophylaxe von Krankheiten.
In einer bevorzugten Ausführungsform betrifft die vorliegende Erfindung die genannten Verbindungen zur Verwendung bei der Behandlung und/oder Prophylaxe von onkologischen Erkrankungen und Tumorerkrankungen jeglicher Pathogenese.

In einer weiteren bevorzugten Ausführungsform betrifft die vorliegende Erfindung die genannten Verbindungen zur Verwendung bei der Behandlung und/oder Prophylaxe der Leishmaniose, Trypanosomiasis und/oder der Pneumocystis carinii Pneumonie (PcP).

In einer weiteren bevorzugten Ausführungsform betrifft die vorliegende Erfindung die genannten Verbindungen zur Verwendung bei der Behandlung und/oder Prophylaxe der Malaria.

Ferner betrifft die vorliegende Erfindung auch ein Arzneimittel umfassend mindestens eine der genannten Verbindungen der Formel (I) gegebenenfalls in Kombination mit einem oder mehreren inerten, nicht-toxischen, pharmazeutisch geeigneten Hilfsstoffen.

Die vorliegende Erfindung betrifft darüber hinaus auch ein Arzneimittel umfassend mindestens eine der genannten Verbindungen der Formel (I) in Kombination mit einem oder mehreren weiteren Wirkstoffen.

Die vorliegende Erfindung betrifft darüber hinaus auch ein Arzneimittel zur oralen oder parenteralen Anwendung.

Die vorliegende Erfindung betrifft des Weiteren auch ein Arzneimittel zur Behandlung und/oder Prophylaxe von onkologischen Erkrankungen und Tumorerkrankungen.

Die vorliegende Erfindung betrifft darüber hinaus auch ein Arzneimittel wie oben beschrieben, dass magensaftresistent formuliert ist.

Darüber hinaus betrifft die vorliegende Erfindung ein Verfahren zur Behandlung und/oder Prophylaxe von Tumorerkrankungen bei Menschen oder Tieren unter Verwendung mindestens einer der genannten Verbindungen der Formel (I) oder eines der genannten Arzneimittel.

Des Weiteren betrifft die vorliegende Erfindung ein Verfahren zur Behandlung und/oder Prophylaxe von Leishmaniose, Trypanosomiasis und der Pneumocystis carinii Pneumonie (PcP).

Die vorliegende Erfindung betrifft auch ein Verfahren zur Herstellung einer Verbindung wie oben beschrieben, bei dem das Amidoxim der Formel (A) 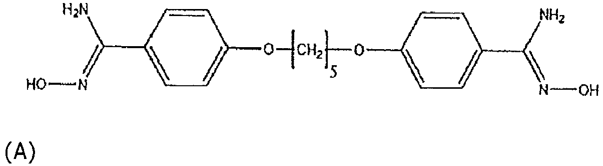 durch Reaktion mit einem Dicarbonsäureanhydrid der Formel (B) 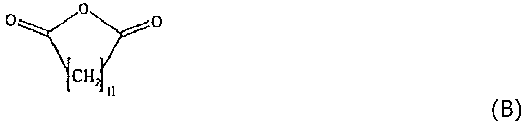 in welcher n für 2 steht in eine Verbindung der Formel (C) 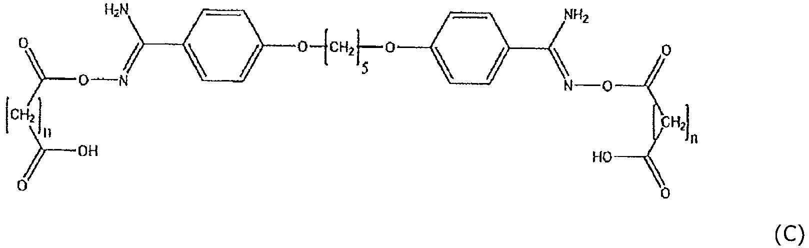 überführt wird.

Ein weiter entwickeltes Prodrug-Prinzip ist die Kopplung von Amidoximen mit Dicarbonsäuren, wie es in den Patentanmeldungen WO2009095499 und DE102008007381 beschrieben ist.¹¹ Bezug nehmend auf diese Arbeiten wurden entsprechende Prodrugs des Pentamidins entwickelt. Die erhaltenen Verbindungen wurden ausführlich charakterisiert und hinsichtlich ihrer Bioverfügbarkeit untersucht. Unsere Studien zeigten, dass es sich bei den Pentamidin-Dicarbonsäure-Derivaten, um besonders geeignete Pentamidin-Prodrugs handelt, die neben einer ausgezeichneten Löslichkeit auch eine gute Bioverfügbarkeit nach oraler Gabe besitzen. Vergleichende Untersuchungen mit anderen Pentamidin-Prodrugs zeigten dabei die Überlegenheit des N,N'bis(succinyloxy)pentamidins **(1)** gegenüber den bisher beschriebenen Pentamidin-Prodrugs.

### Beschreibung der Erfindung

Bisher ist die therapeutische Verwendung des Pentamidins aufgrund der mangelnden oralen Bioverfügbarkeit sehr eingeschränkt. Gerade in den strukturell schwachen Ländern der dritten Welt stellt die Entwicklung eines oral bioverfügbaren Arzneistoffs einen erheblichen Fortschritt in der Pharmako-Therapie dar, da hierdurch die aufwendigen und risikobehafteten intravenösen Applikationen umgangen werden können. Zudem sind die heutigen Behandlungsmöglichkeiten gerade bei Trypanosomen-, Pneumocystis carinii, Pneumocystis jirovecii und Leishmanien-Infektionen nicht zufriedenstellend. Aus diesem Grund ist der Schwerpunkt dieser Erfindung die Entwicklung eines oral bioverfügbaren Prodrugs des Pentamidins.

Zudem könnte ein oral anwendbares Pentamidin-Prodrug erhebliche Bedeutung in der Krebs-Therapie erlangen. Derzeit wird das Pentamidin in klinischen Studien gegen verschiedene Krebs-Arten (Brust- und Kolon-Karzinom) untersucht. Erste klinische Studien zeigten bereits aussichtsreiche Ergebnisse.³ Auch hier könnten die neuen Pentamidin-Prodrugs Einsatz finden und die Therapie verbessern, auch in Kombination mit anderen onkologischen Wirkstoffen.

Im Rahmen dieser Erfindung wurden neuartige Pentamidin-Prodrugs durch Verknüpfung des Pentamidin-Diamidoxims **(3)** mit Dicarbonsäuren entwickelt. Die erhaltenen Verbindungen wurden ausführlich in vitro und in vivo charakterisiert, wobei sie eine ausgezeichnete Löslichkeit sowie gute Bioverfügbarkeit zeigten. Vergleichende Untersuchungen mit anderen Pentamidin-Prodrugs zeigten zudem die Überlegenheit des neu-entwickelten N,N'bis(succinyloxy)pentamidins **(1)** gegenüber bisher beschriebenen Pentamidin-Prodrugs.

### Synthese

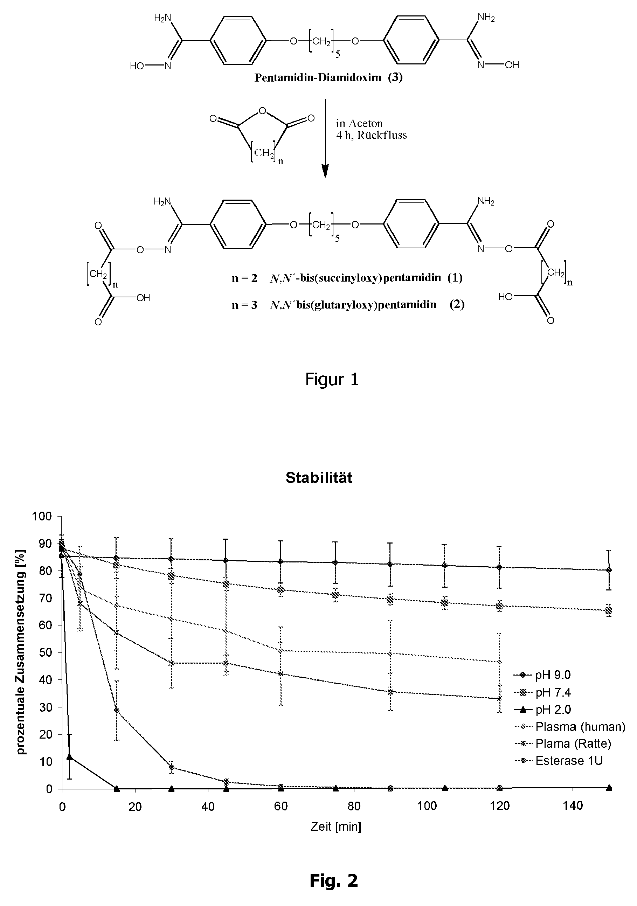

Die Darstellung der Prodrugs (**1**, **2**) erfolgte aus dem Pentamidin-Diamidoxim (**3**) und dem jeweiligen Säureanhydrid (Bernsteinsäure-, bzw. Glutarylsäure-Anhydrid). Die Ausgangsverbindung wurde in getrocknetem Aceton durch Zugabe von Bernsteinsäureanhydrid unter Rückfluss 4 h erhitzt (s. Abbildung 1). Durch anschließendes Aufkochen in Toluol und direktes Abfiltrieren konnten die Substanzen **1** und **2** abgetrennt und die gewünschten Verbindungen analysenrein dargestellt werden.

### Stabilität

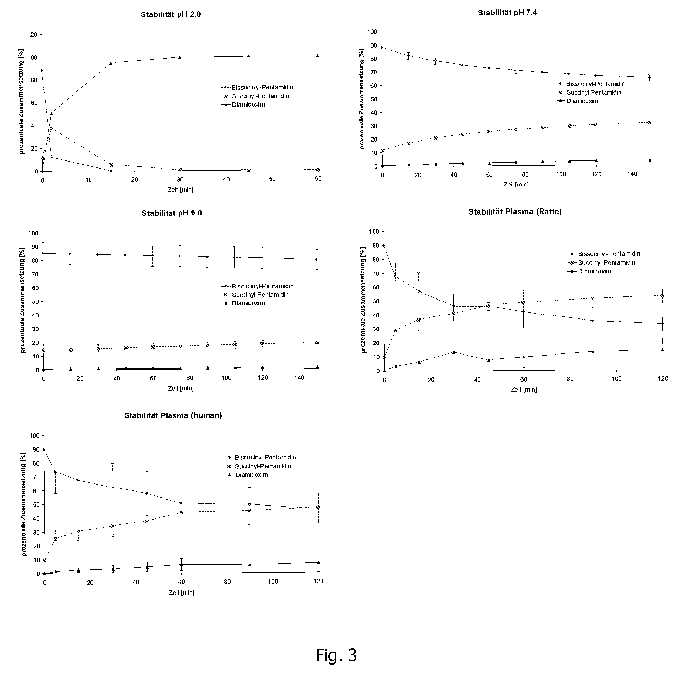

Die Untersuchungen zeigten, dass die Verbindung **1** stabil im neutralen und leicht alkalischen pH-Bereich, also pH 7,4 bis pH 9,0, ist. Im sauren Milieu bei pH 2.0 werden die Verbindungen rasch hydrolytisch gespalten (Abbildung 2, 3).

Bei den Untersuchungen zeigte sich, dass das N,N'-bis(succinyloxy)pentamidin **(1)** in wässrigem Milieu in Mono-Succinylpentamidin und Pentamidin-Diamidoxim **(3)** hydrolysiert. Während diese Hydrolyse bei pH 7.4 und pH 9.0 nur in geringem Ausmaß abläuft, erfolgt sie bei pH 2.0 und in humanem sowie murinem Plasma ausgeprägt. Die rasche Hydrolyse des N,N'-bis(succinyloxy)pentamidins **(1)** bei pH 2.0 (s. Abbildung 2, 3) ist hinsichtlich der Verwendung als Prodrug als problematisch einzustufen. Das N,N'-bis(succinyloxy)pentamidin **(1)** würde nach oraler Applikation im sauren Milieu des Magens zu einer schnellen Hydrolyse des Prodrugs zum Pentamidin-Diamidoxim **(3)** führen. Da der Hauptanteil der gastrointestinalen Absorption jedoch erst in den oberen Dünndarmabschnitten erfolgt, ist eine magensaftresistente Formulierung dieses Prodrugs anzustreben. Auf diese Weise würde das Prodrug das saure Milieu des Magens unbeschadet überstehen und könnte später im Dünndarm absorbiert werden. Die Instabilität bei pH 2.0 ist daher für die spätere Verwendung als Arzneistoff als unproblematisch einzustufen.

### Löslichkeit

Das N,N'-bis(sucinyloxy)pentamidin **(1)** besitzt eine sehr gute Löslichkeit im pH Bereich von 7.4 bis 9.0 (s. Tabelle 1). Die Löslichkeit im sauren Milieu (pH 2.0) konnte aufgrund der zuvor beschriebenen Hydrolyse in diesem Milieu nicht exakt charakterisiert werden. Experimente zeigten allerdings auch hier, dass die Löslichkeit im mM-Bereich liegt.

Tabelle 1 zeigt die Löslichkeit des N,N'-bis(succinyloxy)pentamidins **(1)** im Vergleich zu anderen entwickelten Pentamidin-Prodrugs. Aus diesen Daten wird deutlich, dass es sich bei dem Dicarbonsäure-Derivat **(1)** um die Verbindung mit der besten Löslichkeit handelt. Einzig das Pentamidin-Monoamidoxim ist ebenfalls im mM-Bereich bei neutralem und leicht alkalischem pH-Wert löslich. Diese Verbindung besitzt jedoch noch eine freie Amidinfunktion, welche eine sehr nachteilige Wirkung auf die orale Bioverfügbarkeit besitzt. Diese ausgezeichneten Löslichkeitseigenschaften begünstigen eine spätere Verwendung als Arzneistoff, da eine ausreichende Löslichkeit eine Grundvoraussetzung für eine ausreichende orale Absorption darstellt. Zusätzlich sind durch die gute Löslichkeit des N,N'-bis(succinyloxy)pentamidins **(1)** auch parenterale Applikationsformen möglich, wie Injektionen oder Infusionen.

### Proteinbindung

Die Untersuchungen zur Plasmaproteinbindung zeigten, dass diese Verbindung mit einer Plasmaproteinbindung von 97% über eine recht ausgeprägte Proteinbindung verfügt. Die ermittelte Proteinbindung liegt in dem Bereich, der auch bei anderen Pentamidin-Prodrugs beschrieben ist und stellt daher keinen Nachteil im Vergleich zu den anderen Prodrugs dar.⁹

### Prodrug-Konzept

Das den erfindungsgemäßen Verbindungen zugrundeliegende Prodrug-Konzept selbst wurde in den Patentanmeldungen WO2009095499 undDE102008007381beschrieben.

Die Aktivierung der erfindungsgemäßen Prodrugs verläuft über Esterasen und das mARC-Enzymsystem und ist somit unabhängig von Cytochrom P450-Enzymen. Die Beteiligung von P450 Enzymen birgt stets das Risiko von Interaktionen, welche bei dem von uns gewählten Aktivierungsmechanismus nicht beschrieben sind. Cytochrom P450 Enzyme sind an der Metabolisierung zahlreicher Arzneistoffe beteiligt. Werden mehrere Arzneistoffe eingenommen, welche über dieses Enzymsystem verstoffwechselt werden, kann es zu einer Verzögerung des Abbaus der Arzneistoffe mit klinisch relevanten Nebenwirkungen kommen.

### In vitro Aktivierung

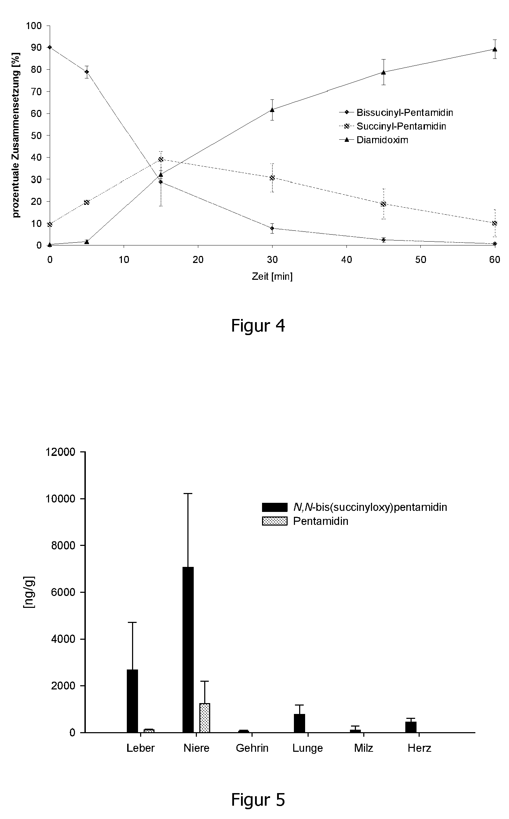

Die durchgeführten in vitro Aktivierungsstudien zeigten, dass die Aktivierung des N,N'-Bis(succinyloxy)pentamidins **(1)** in einem guten Ausmaß erfolgt (Tabelle 2). Die Inkubation mit Carboxyl-Esterasen aus Schweineleber führte zur einer raschen Aktivierung des N,N'-bis(succinyloxy)pentamidins **(1)** (s. Abbildung 4). Bereits nach einer Inkubationszeit von 60 min waren ca. 90 % des eingesetzten Substrates aktiviert. Dieses Ergebnis zeigt, dass der erste Schritt der Aktivierung des N,N'-bis(succinyloxy)pentamidin **(1)** zum Diamidoxim mit hervorragender Geschwindigkeit abläuft.

Bei den Inkubationen mit subzellulären Enzympräparationen konnte die Reduktion zum Pentamidin nachgewiesen werden (Tabelle 2). Generell sind die Enzymquellen porcinen Ursprungs aktiver als die humanen, was mit der Art der Gewinnung der Enzympräparationen zu begründen. Es ist zu berücksichtigen, dass die Aufarbeitung von humanen Organen aufgrund sehr geringer Ausgangsmengen problematischer ist. Zudem stammen die Schweineorgane in der Regel von gesunden Tieren, wohingegen humane Gewebeproben meist Karzinom-Patienten nach Organresektion entnommen werden, was eine Erklärung für die vergleichsweise niedrigen Umsetzungsraten bei Verwendung humaner Enzympräparationen darstellt.

Zusammenfassend kann festgehalten werden, dass es sich bei dem N,N'-bis(succinyloxy)pentamidin **(1)** um ein geeignetes Prodrug des Pentamidins handelt. Diese Studie erweist den generellen Nachweis, dass die Bioaktivierung der Prodrugs zur aktiven Verbindung abläuft. Es ist zu erwarten, dass die Umsetzungsraten in vivo deutlich höher liegen, da die benötigten Enzyme in höheren Mengen zur Verfügung stehen.

### Orale Bioverfügbarkeit

In den durchgeführten Tierstudien konnte die orale Bioverfügbarkeit des N,N'-bis(succinyloxy)pentamidins **(1)** demonstriert werden. Nach oraler Applikation des Prodrugs konnten keine Pentamidin-Plasmaspiegel detektiert werden, was mit der bekannt hohen Akkumulationsneigung des Pentamidins in Organen zu begründen ist. Die Analyse der Organproben zeigte, dass N,N'-(bissuccinyloxy)pentamidin **(1)** oral bioverfügbar ist. Es konnten nach oraler Applikation des Prodrugs relevante Konzentrationen in allen untersuchten Organen (Leber, Niere, Lunge, Herz, Gehirn und Milz) nachgewiesen werden. Die höchsten Konzentrationen wurden dabei in Niere und Leber nachgewiesen (Abbildung 5). Die Konzentrationen in Milz, Herz, Gehirn und Lunge waren deutlich geringer. Die relative orale Bioverfügbarkeit konnte je nach Organ mit bis zu 98% bestimmt werden (Tabelle 3).

Zusammengefasst belegen die Daten die hervorragende Eignung des erfindungsgemäßen Prodrug-Prinzips für das Pentamidin. Die in den Organen nachgewiesenen Pentamidin-Konzentrationen liegen in einem Bereich, der die Therapie von Infektionen sowohl mit Trypanosomen (IC₅₀: 0.8 - 3.2 nM), Leishmanien (IC₅₀: 820 - 2590 nM) als auch mit Plasmodien (IC₅₀: 35 - 129 nM) ermöglicht.¹³⁻¹⁶

### Zusammenfassung

Bei den neu entwickelten Prodrugs handelt es sich um oral bioverfügbare Prodrugs des Pentamidins. Das verwendete Prodrug-Prinzip führt zu einer erheblichen Verbesserung der Löslichkeit, die einen sehr kritischen Parameter anderer Pentamidin-Prodrugs darstellt. Durch diese verbesserte Löslichkeit wird das pharmakokinetische Verhalten der Substanz positiv beeinflusst, da gute Lösungseigenschaften einen wichtigen Parameter bei der Absorption von Arzneistoffen, insbesondere im Gastroinestinaltrakt darstellen.

Die Verbindung **1** besitzt mit Ausnahme des sauren pH-Bereichs eine gute chemische Stabilität. Die ausgeprägte Hydrolyse im sauren Milieu führt dazu, dass das Prodrug bei der oralen Applikation als magensaft-resistente Formulierung verabreicht werden sollte, um eine Hydrolyse im Magen auszuschließen.

In den in vitro Bioaktivierungs-Assays konnte eine rasche und umfangreiche Aktivierung des Prodrugs zum Pentamidin nachgewiesen werden. Die Aktivierung verläuft unabhängig von Cytochrom P450-Enzymen und birgt somit nicht das Risiko von Interaktionen.

In den abschließend durchgeführten Tierstudien konnte die gute orale Bioverfügbarkeit auch experimentell belegt werden. Die in den Organen detektierten Gehälter an Pentamidin sind in einem Bereich, der eine Wirksamkeit gegenüber Infektionen durch Trypanosomen, Leishmanien und Plasmodien ermöglicht.

Zusammengefasst handelt es sich bei den Pentamidin-Dicarbonsäure-Derivaten um ausgezeichnete Prodrugs, die über exzellente physikochemische Parameter verfügen und eine gute orale Bioverfügbarkeit besitzen. Aufgrund dieser Eigenschaften sind sie anderen Pentamidin-Prodrugs deutlich überlegen. Ein Einsatz ist sowohl in der Krebs-Therapie als auch bei der Behandlung von Trypanosomen-, Leishmanien- und Pneumocystis carinii Infektionen möglich.

Die beschriebene Erfindung wird durch die beigefügten Figuren noch näher erläutert.
Figur 1: Syntheseschema der Pentamidin-Prodrugs
Figur 2: Stabilität von N,N'-bis(succinyloxy)pentamidin **(1)** bei verschiedenen pH-Werten und in murinem bzw. humanem Plasma, sowie bei Inkubation mit Esterase.
Figur 3: Stabilität von N,N'-bis(succinyloxy)pentamidin **(1)** bei verschiedenen pH-Werten und in murinem bzw. humanem Plasma.
Figur 4: Aktivierung des N,N'-bis(succinyloxy)pentamidins **(1)** durch Esterasen.
Figur 5: Gehalt an Pentamidin nach p.o.-Applikation (50 mg/kg) von Pentamidin und N,N-bis(succinyloxy)pentamidin **(1)** in Organen. Dargestellt sind die Mittelwerte aller untersuchten Ratten.

### Material und Methoden: Ausführungsbeispiele Synthesen

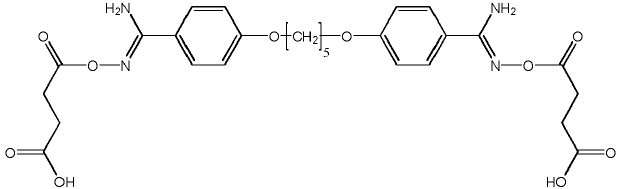

### 4,4'-Pentamethylendioxy-bis-[N-(carboxypropionyloxy)]benzamidin (N,N'-bis(succinyloxy)pentamidin) (1):

1 g Pentamidin-Diamidoxim wird in 250 ml Aceton gelöst und 540 mg Bernsteinsäure-Anhydrid zugefügt. Der Ansatz wird unter Rückfluss für 4 h gerührt. Anschließend wird das Lösemittel in Vakuum entfernt und der Rückstand aus Toloul kristallisiert.
Ausbeute: 68%
Schmelzpunkt: 141°C
IR (KBr):
   v^{~}= 3478, 3348, 2940, 2870, 1732, 1698, 1612, 1472, 1250 cm⁻¹
¹H NMR (DMSO-d₆):
   δ/ppm (TMS) = 1.59 (m, 2H, CH₂), 1.79 (qn, 4H, ³J = 6.7 Hz, CH₂), 2.52 (t, 4H, ³J = 6.6 Hz, CH₂), 2.68 (t, 4H, ³J = 6.6 Hz, CH₂), 4.04 (t, 4H, ³J = 6.5 Hz, O-CH₂), 6.63 (s, 4H, NH₂), 6.99 (m_{c}, 4H, AA'BB', Ar-H), 7.65 (m_{c}, 4H, AA'BB', Ar-H), 12.18 (brs, 2H, COOH)
¹³C-NMR (DMSO-d₆):
   δ/ppm (TMS) = 22.1 (CH₂), 27.9 (CH₂), 28.3 (CH₂), 28.8 (CH₂), 67.5 (O-CH₂), 113.9 (ArCH), 123.5 (ArC), 128.1 (ArCH), 156.2 (ArC), 160.3 (C-NH₂), 170.2 (COOR), 173.5 (COOH)
MS (ESI) m/z:
   573 [M+H]⁺, 555 [M-H₂O+H]⁺, 473 [M-C₄H₄O₃+H]⁺, 455 [M-C₄H₄O₃-H₂O+H]⁺, 373 [DAO+H]⁺, 178
Elementar-Analyse C₂₇H₃₂N₄O₁₀ (molekulare Masse: 572.56):
   Berechnet: C 56.64, H 5.63, N 9.79
   Gefunden: C 56.85, H 6.01, N 9.60

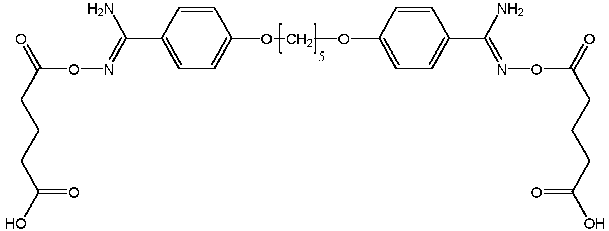

### 4,4'-Pentamethylendioxy-bis-[N-(carboxybutionyloxy)]benzamidin (N,N'-bis(glutaryloxy)pentamidin) (2):

1 g Pentamidin-Diamidoxim wird in 250 ml Aceton gelöst und 616 mg Glutarsäure-Anhydrid zugefügt. Der Ansatz wird unter Rückfluss für 4 h gerührt. Anschließend wird das Lösemittel in Vakuum entfernt und der Rückstand aus Toloul kristallisiert.
Ausbeute: 80%
Schmelzpunkt: 155°C
IR (KBr):
   v^{~} = 3495, 3350, 2950, 2874, 1747, 1700, 1619, 1520, 14225, 1258 cm⁻¹
¹H NMR (DMSO-d₆):
   δ/ppm (TMS) = 1.59 (m, 2H, CH₂), 1.81 (m, 8H, CH₂), 2.29 (t, 4H, ³J = 7.4 Hz, CH₂), 2.49 (t, 4H, ³J = 7.1 Hz, CH₂), 4.04 (t, 4H, ³J = 6.4 Hz, O-CH₂), 6.63 (s, 4H, NH₂), 6.98 (m, 4H, AA'BB', Ar-H), 7.65 (m, 4H, AA'BB', Ar-H), 12.05 (s, 2H, COOH)
¹³C-NMR (DMSO-d₆):
   δ/ppm (TMS) = 19.9 (CH₂), 22.1 (CH₂), 28.3 (CH₂), 31.6 (CH₂), 32.8 (CH₂), 67.5 (O-CH₂), 114.1 (ArCH), 123.5 (ArC), 128.1 (ArCH), 156.1 (ArC), 160.3 (C-NH₂), 170.6 (COOR), 173.9 (COOH)
MS (ESI) m/z:
   601 [M+H]⁺, 169
Elementar-Analyse C₂₉H₃₆N₄O₁₀ (molekulare Masse: 600.62):
   Berechnet: C 57.99, H 6.04, N 9.33
   Gefunden: C 58.05, H 6.24, N 9.72

### Charakterisierung der Pentamidin-Prodrugs

### Stabilitätsuntersuchungen des N,N'-bis(succinyloxy)pentamidins (1)

Für die Stabilitätsuntersuchungen wurden eine 0.1 mM Lösung des N,N'-bis(succinyloxy)pentamidin **(1)** in 50 mM Kaliumphosphat-Puffer / DMSO (90/10, vol/vol) hergestellt. Die Untersuchung erfolgte bei den pH-Werten 2.0, 7.4 und 9.0. Alle 15 min wurden über einen Zeitraum von 150 min eine Probe gezogen und umgehend per HPLC analysiert.

Weitere Untersuchungen wurden mit humanem und murinem Plasma durchgeführt. Es wurden 900 µl des Plasmas mit 100 µl einer 2 mM N,N'-bis(succinyloxy)pentamidin **(1)** Lösung versetzt. Somit betrug die Endkonzentration an N,N'-bis(succinyloxy)pentamidin **(1)** 0.2 mM. Die Proben wurden bei 37°C im Wasserschüttelbad inkubiert und nach 0, 15, 30, 45, 60, 75, 90, 105 und 120 min Proben entnommen. Dazu wurden jeweils 100 µl entnommen und mit 100 µl Acetonitril versetzt. Die Proben wurden geschüttelt, für 5 min zentrifugiert und der Überstand mit der HPLC vermessen.

Zusätzlich wurden Inkubationen mit Carboxyl-Esterase aus Schweineleber durchgeführt. Dazu wurde das N,N'-bis(succinyloxy)pentamidin **(1)** in einer Konzentration von 0.1 mM mit 1 U Esterase in 250 µl 50 mM Phosphatpuffer pH 7.4 bei 37°C über einen Zeitraum von 60 min inkubiert. In Zeitabständen von jeweils 15 min wurden die Proben mit Hilfe der HPLC analysiert.

Die Stabilitätsuntersuchungen wurden mit Hilfe folgender HPLC-Methode ausgewertet:

| | | | |
|---|---|---|---|
| HPLC-System | Waters Alliance™ HPLC-System mit Waters e2695 XC Separations Modul, Waters 2998 Photodiode Array Detector und Empower™ 2 Aufnahme- und Auswertesoftware | | |
| Stationäre Phase | Synergi Max-RP 80A (Phenomenex, 250 x 4.6 mm; 4 µm) mit einer Phenomenex C18 (4 x 3.0 mm) Vorsäule | | |
| Mobile Phase | A | 45% | 20 mM Phosphatpuffer pH 7.0 |
| | B | 55% | Methanol |
| Detektion | 210 - 400 nm (260 nm) | | |
| Flussrate | 1.0 ml/min | | |
| Laufzeit | 12 min | | |
| Säulentemperatur | 25°C | | |
| Injektionsvolumen | 10 µl | | |
| Retentionszeiten | N,N'-bis(succinyloxy)pentamidin (**1**): | | 3.2 ± 0.1 min |
| | Succinyloxypentamidin: | | 4.8 ± 0.1 min |
| | Pentamidin-Diamidoxim (**3**): | | 8.1 ± 0.2 min |

### Löslichkeit von N,N'-bis(succinyloxy)pentamidin (1)

Eine in 100 µl unlösliche Menge der Verbindung wurde in 50 mM Phosphatpuffer (pH 7.4 bzw. 9.0) suspendiert und für 20 min geschüttelt. Anschließend wurde der ungelöste Teil abzentrifugiert (12.000 rpm) und die Proben sofort per HPLC vermessen. Die Auswertung der Löslichkeit erfolgte über eine Kalibrierung von N,N'-bis(succinyloxy)pentamidin **(1)** in DMSO. Bei einem physiologischen pH-Wert von 7.4 ist die Verbindung gut löslich (7.5 mM). Die Löslichkeit wird weiter verbessert, wenn der pH-Wert erhöht wird (s. Tabelle 1).

Als Vergleich wurden verschiedene andere Pentamidin-Prodrugs untersucht, um die Löslichkeit im Vergleich bereits beschriebener Derivate besser beurteilen zu können. Die Bestimmung der Löslichkeiten wurde analog der für Verbindung **1** beschriebenen Methode durchgeführt.

**Tabelle 1: Löslichkeit des N,N'-bis(succinyloxy)pentamidins (1) und anderer Pentamidin-Prodrugs bei unterschiedlichen pH-Werten**

| **Pentamidin-Prodrug** | **Löslichkeit [µM]** | | |
|---|---|---|---|
| | **pH 2.0** | **pH 7.4** | **pH 9.0** |
| ***N,N'*-bis(succinyloxy)pentamidine (1)** | Hydrolyse | 7500 ± 340 | 10780 ± 70 |
| **Pentamidin-Monomamidoxim** | 22285 ± 1244 | 1370 ± 291 | 1257 ± 40 |
| **Pentamidin-Diamidoxim (3)** | 4211 ± 231 | 12 ± 1 | 4 ± 1 |
| ***N,N*'-bis(acetoxy)pentamidine** | 14 ± 8 | 2 ± 1 | 3 ± 2 |
| ***N,N'*-bis(methoxy)pentamidine** | 1304 ± 28 | 8 ± 1 | 10 ± 2 |
| ***N,N'*-bis(dihydroxy)pentamidine** | > 35000 | 95 ± 8 | 21 ± 3 |
| ***N,N'*-bis(valoxy)pentamidine** | > 35000 | 157 ± 19 | 84 ± 18 |

### Bestimmung der Proteinbindung des N,N'-bis(succinyloxy)pentamidins (1)

Die Plasmaprotein-Bindung wurde in drei verschiedenen Konzentrationen (10, 20 und 50 µM) durchgeführt. Als Proteinlösungen wurde eine 4%ige Albumin-Lösung verwendet. Es wurden jeweils 50 µl einer 10fach konzentrierten Substanz-Lösung in 450 µl der Proteinlösung pipettiert. Die Inkubation erfolgte über 15 min im Schüttelwasserbad bei 37°C. Im Anschluss wurden die Proben in Ultrafiltrationseinheiten (Vivaspin 500, 10 kDa cut off) überführt und für 15 min bei 10.000 RPM zentrifugiert. Das Filtrat wurde per HPLC analysiert. Zusätzlich wurde für jede Konzentration eine Kontrolle durchgeführt, die nicht mit Protein versetzt und nicht zentrifugiert wurde. Eine weitere Kontrolle ohne Proteinzusatz, die jedoch durch die Filtrationseinheit abzentrifugiert wurde, zeigte, dass das die Prodrugs nicht von der Membran zurückgehalten werden und dienten der Validierung der Methodik.

Die Analyse der Probe ergab eine Proteinbindung der Verbindung **1** von 97.1 ± 1.2 %.

### Untersuchung der Bioaktivierung des N,N'-bis(succinyloxy)pentamidins (1)

### Ermittlung der Aktivierung der Prodrugs mit verschiedenen subzellulären Enzymsystemen:

Die Aktivierung des Prodrugs wurde in vitro mittels subzellulärer Enzympräparationen bestimmt. Als Enzympräparationen wurden 9000xg Überstände, Mikrosomen und Mitochondrien aus humanen und porcinen Leber-und Nierengeweben verwendet. Die Inkubationsansätze setzten sich aus 500 µM Prodrug, 1 mM NADH, 1 U Esterase und 0.3 mg Enzympräparation, gelöst in 150 µl 100 mM Phosphatpuffer pH 6.3, zusammen. Die Inkubation erfolgte über 20 min bei 37°C im Schüttelwasserbad. Beendet wurde die Inkubation durch Zugabe von 150 µl Acetonitril. Anschließend wurden die Proben 10 min geschüttelt und das ausgefällte Protein bei 10.000 RPM für 15 min abzentrifugiert. Der Überstand wurde mit Hilfe der HPLC vermessen. Die ermittelten Umsetzungsraten sind in Tabelle 2 aufgeführt.

**Tabelle 2: Aktivierung des N,N'-bis(succinyloxy)pentamidins (1) in die Wirkform mit subzellularen Enzympräparationen, HL = humane Leber, HN = humane Niere, SL = Schweineleber, SN = Schweineniere, 9000g = 9000g Überstand, MS = Mikrosomen, Mt = Mitochondrien**

| **Enzymquelle** | **Pentamidin [nmol*min⁻¹*mg⁻¹]** |
|---|---|
| **HL 9000g** | 0.04± 0.01 |
| **HL Ms** | 0.02± 0.02 |
| **HL Mt** | 0.56± 0.43 |
| **HN Mt** | 0.08± 0.02 |
| **SL 9000g** | 0.00± 0.00 |
| **SN 9000g** | 0.49± 0.03 |
| **SL Ms** | 0.69± 0.13 |
| **SN Ms** | 2.25± 0.58 |
| **SL Mt** | 1.44± 0.22 |
| **SN Mt** | 0.41 ± 0.09 |

Zusätzlich wurden Inkubationen mit 1 U Carboxylesterase aus Schweinleber durchgeführt. Dazu wurde die Verbindung in einer Konzentration von 500 µM mit 1 U Esterase in 250 µl 50 mM Phosphatpuffer pH 7.4 über 60 min inkubiert. Die Inkubationen wurden durch Zugabe von 250 µl Acetonitril beendet. Die Inkubationen mit Carboxyl-Esterasen aus Schweineleber führte zur einer raschen Aktivierung des N,N'-bis(succinyloxy)pentamidins **(1)** (s. Abbildung 4). Bereits nach einer Inkubationszeit von 60 min waren ca. 90 % des eingesetzten Substrates aktiviert. Dieses Ergebnis zeigt, dass der erste Schritt der Aktivierung des N,N'-bis(succinyloxy)pentamidins **(1)** zum Diamidoxim mit großer Geschwindigkeit abläuft.

**HPLC-Methode zur Bestimmung des Pentamidins**

| | | |
|---|---|---|
| HPLC-System | Waters Alliance HPLC-System mit Waters e2695 XC Separations Modul, Waters 2998 Photodiode Array Detector und Empower 2 Software | |
| Säule: | LiChroCart LiChrospher 60 RP-select B, 125 x 4 mm, 5 µm | |
| Fluss: | 1 ml/min | |
| Fliessmittel: | 52% | 20 mM Tetramethylammoniumchlorid / 10 mM Octylsulfonat pH 3.0 |
| | 48% | MeOH |
| Laufzeit: | 15 min | |
| Detektion: | 260 nm | |
| Injektionsvolumen: | 20 µl | |
| Retentionszeit: | | Pentamidin 10.7 ± 0.4 min |

### Orale Bioverfügbarkeit (Tierstudie)

Pentamidin wurde 10 Ratten in einer Konzentration von 10 mg/kg intravenös verabreicht. Das N,N'-bis(succinyloxy)pentamidin **(1)** wurde 10 Ratten in einer Konzentration von 50 mg/kg als Suspension mit Arabischem Gummi (10% m/V) per Schlundsonde appliziert. Zur Herstellung der Suspension wurde 100 mM Kaliumphosphat-Puffer pH 9.0 verwendet, um eine vorzeitige Abspaltung der Succinylester im sauren Milieu des Magens zu verhindern. Zusätzlich wurde 3 Ratten Pentamidin in einer Dosierung von 50 mg/kg per Schlundsonde appliziert, um die orale Bioverfügbarkeit der Wirkform selbst zu bestimmen.

Nach i.v.-Gabe wurden Plasmaproben nach 5, 10, 20, 40, 75, 150 und 300 min abgenommen bzw. nach oraler Applikation nach 20, 40, 60, 90, 120, 240 und 360 min. Hierzu wurden 300 µl Vollblut mit einer Insulinspritze entnommen und in EDTA beschichtete Microvetten CB 300 (Sarstedt, Nümbrecht) überführt. Nach jeder Abnahme wurde mit 100 µl 0,9%-iger Kochsalzlösung bzw. im Abstand von 60 min mit Heparin-Lösung (250 I.E./ml) gespült. Die Blutprobe wurde kurz geschüttelt und bis zur Zentrifugation (4°C; 14000 U/min; 10 min) auf Eis gestellt. Die weitere Lagerung der Proben erfolgte bei -80°C.

Die Tötung erfolgte durch Dekapitation 6 Stunden nach Medikamentengabe mit einer Guillotine. Im Anschluss wurden die Organe entnommen. Alle Organe wurden gesäubert und in mit Trockeneis gekühltem 2-Methylbutan gefroren. Es wurden Leber, Niere, Lunge, Milz, Herz und Gehirn entnommen.

### Probenaufarbeitung

### 1. Plasmaproben

Die Plasmaproben wurden bei Raumtemperatur aufgetaut. Es wurden jeweils 65 µl Acetonitril vorgelegt und 65 µl der Plasmaproben dazu pipettiert. Anschließend wurden die Proben für 45 min geschüttelt. Die Proben wurden für 15 min bei 10.000 RPM zentrifugiert und der Überstand in HPLC-Vials überführt. Jeweils 35 µl wurden für die Bestimmungen mittels HPLC verwendet.

Zur quantitativen Auswertung der Plasmaproben wurden Kalibrierungen und Wiederfindungen des Pentamidins in Phosphatpuffer pH 7.4 bzw. in murinem Plasma durchgeführt bzw. bestimmt.

### 2. Organproben

Die Organe wurden bei Raumtemperatur aufgetaut und gewogen. Abhängig vom jeweiligen Organ wurden unterschiedliche Mengen Gewebe aufgearbeitet. Bei den Leberproben wurden ca. 1000 mg verwendet; bei allen anderen Organen ca. 500 mg. Die Organe wurden mit Hilfe eines Potters zerkleinert. Dazu wurden die abgewogenen Gewebe mit je 1 ml Aqua bidest. für 5 min zerkleinert. Anschließend wurde mit jeweils 1 ml Aqua bidest das Potter-Gefäß ausgespült. Die Proben wurden in Reaktionsgefäße überführt und das gleiche Volumen Acetonitril zugesetzt, um Proteine auszufällen. Die Proben wurden 45 min geschüttelt und anschließend für 15 min bei 12.000 RPM zentrifugiert. Der Überstand wurde in Glasflaschen überführt und unter Druckluft einkonzentriert. Der Rückstand wurde mit 500 µl Acetonitril gewaschen, erneut zentrifugiert und der Überstand den restlichen Proben zugesetzt. Der Rückstand wurde verworfen. Nach dem Einkonzentrieren unter Druckluft wurden die Proben über Nacht gefriergetrocknet.

Das Anlösen der Proben erfolgt mit 400 µl eines Gemisches aus Methanol/Aqua bidest. (50/50). Die Proben wurden für 1.5 Stunden bei Raumtemperatur geschüttelt und anschließend der Rückstand abzentrifugiert (15.000 RPM, 15 min). Die Pentamidin-Konzentration wurde aus dem Überstand mittels HPLC bestimmt.

### Ergebnisse der Tierstudie

Die Analyse der Plasmaproben nach intravenöser Applikation des Pentamidins lieferte detektierbare Plasmaspiegel über einen Zeitraum von 300 min. Nach oraler Applikation des Prodrugs konnten keine Plasma-Konzentrationen von Pentamidin detektiert werden. Dieses Phänomen ist für Pentamidin-Derivate bekannt, da sie in einem sehr ausgeprägten Ausmaß zur Akkumulation in den Geweben neigen. Eine direkte Berechnung der Bioverfügbarkeit über Plasma-Konzentrationen konnte demnach nicht erfolgen. Zur Bestimmung der relativen Bioverfügbarkeit wurden daher die Pentamidin-Konzentrationen in den untersuchten Organen verwendet.

### Auswertung der Organproben und Bioverfügbarkeit:

Die Analyse der aufgearbeiteten Organproben lieferte detektierbare Gehälter an Pentamidin in allen untersuchten Organen - mit den höchsten Konzentrationen in Leber und Niere. Die Konzentrationen in Lunge, Milz und Herz sind deutlich geringer. Die niedrigsten Konzentrationen an Pentamidin wurden in den Gehirnen detektiert. Die Ergebnisse sind in Abbildung 5 zusammengefasst.

Allgemein wird die orale Bioverfügbarkeit einer Verbindung über die Plasmakonzentrationen nach oraler und intravenöser Applikation der Verbindung bestimmt. Aufgrund der hohen Proteinbindung des Pentamidins und seiner ausgeprägten Neigung zur Akkumulation in Geweben konnten nach oraler Applikation des Pentamidin-Prodrugs jedoch keine Plasmakonzentrationen bestimmt werden. Zur Berechnung der relativen Bioverfügbarkeit werden daher nicht die Plasmakonzentrationen verwendet, sondern die detektierten Gehälter in den untersuchten Organen (Leber, Niere, Lunge, Milz, Herz, Gehirn). Über den Vergleich nach intravenöser Applikation der Wirkform und oraler Applikation des Prodrugs konnte eine relative Bioverfügbarkeit des Pentamidin-Prodrugs berechnet werden. Die unterschiedlichen Dosierungen wurden bei der Berechnung berücksichtigt. Die relativen Bioverfügbarkeiten sind in Tabelle 3 dargestellt. Die höchste Bioverfügbarkeit wurde mit 98% in der Leber nachgewiesen. Die Bioverfügbarkeit in den anderen Geweben ist deutlich reduziert. Die hohe Bioverfügbarkeit in der Leber kann mit der Bioaktivierung des Prodrugs erklärt werden. Sie findet vorrangig in der Leber statt, was die vergleichsweise hohen Konzentrationen in diesem Organ erklärt. Die Konzentration im Gehirn ist sehr gering, was darauf hindeutet, dass das Prodrug die Blut-Hirnschranke nur in sehr geringem Ausmaß überwindet.

**Tabelle 3: Relative Bioverfügbarkeit der Pentamidin-Derivate**

| **Konzentration Pentamidin [µg/g Organ] und relative Bioverfügbarkeit [%]** | | | | | |
|---|---|---|---|---|---|
| | **Pentamidin i.v. (10 mg/kg)** | **Pentamidin p.o. (50 mg/kg)** | **rBV [%]** | **N,N'-bis(succinylo xy)-pentamidin p.o. (50 mg/kg)** | **rBV [%]** |
| **Leber** | 0.53 ± 0.33 | 0.12 ± 0.03 | 4.5 ± 1.1 | 2.68 ± 2.02 | 97.8 ± 73.7 |
| **Niere** | 22.03 ± 4.16 | 1.24 ± 0.96 | 1.1 ± 0.9 | 7.07 ± 3.15 | 6.2 ± 2.8 |
| **Lunge** | 3.03 ± 1.04 | n.d. | - | 0.76 ± 0.42 | 4.9 ± 2.7 |
| **Milz** | 1.97 ± 1.00 | n.d. | - | 0.10 ± 0.16 | 1.0 ± 1.6 |
| **Herz** | 2.41 ± 0.74 | n.d. | - | 0.43 ± 0.16 | 3.5 ± 1.3 |
| **Gehir n** | 0.22 ± 0.12 | n.d. | - | 0.06 ± 0.05 | 5.3 ± 4.4 |

| | | | | | |
|---|---|---|---|---|---|
| rBV = relative Bioverfügbarkeit | | | | | |

### HPLC-Analytiken

Zur Analyse der Organ- und Plasmaproben nach i.v.-Applikation von Pentamidin wurde folgende HPLC-Analytik verwendet:

| | | |
|---|---|---|
| HPLC-System | Waters Autosampler 717plus, Waters 600 Controller, Waters 600 Pump, Waters 2487 Dual λ Absorbance Detector und EZChrom Elite Client/Server Aufnahme-und Auswertesoftware (Version 2.8.3) | |
| Stationäre Phase | Superspher 60 RP-select B (250 x 3 mm); Vorsäule: Merck LiChrospher 60 RP-select B (4 x 4 mm, 5 µm) | |
| Mobile Phase | 40% | Methanol |
| | 60% | TFA 0.1% pH 2.5 |
| Detektion | λ_{Ex} = 275 nm; | λ_{Em} = 340 nm |
| Flussrate | 0.32 ml/min | |
| Laufzeit | 35 min | |
| Injektionsvolumen | 35 µl | |
| Retentionszeit | Pentamidin: | 22.4 ± 1.2 min |

Zur Analyse der Organ- und Plasmaproben nach oraler Applikation der Pentamidin-Prodrugs wurde folgende HPLC-Analytik verwendet:

| | | |
|---|---|---|
| HPLC-System | Waters Alliance™ HPLC-System mit Waters e2695 XC Separations Modul, Waters 2998 Photodiode Array Detector und Empower™ 2 Aufnahme- und Auswertesoftware | |
| Stationäre Phase | Superspher 60 RP-select B (250 x 3 mm); Vorsäule: Merck LiChrospher 60 RP-select B (4 x 4 mm, 5 µm) | |
| Mobile Phase | 40% | Methanol |
| | 60% | TFA 0.1% pH 2.5 |
| Detektion | 210-300 nm (260 nm) | |
| Flussrate | 0.32 ml/min | |
| Laufzeit | 35 min | |
| Injektionsvolumen | 35 µl | |
| Retentionszeit | Diamidoxim | 20.0 ± 0.3 min |
| | Monoamidoxim: | 22.5 ± 0.4 min |
| | Pentamidin: | 24.7 ± 0.5 min |

### Referenzliste:

1. Chow, T. Y.; Alaoui-Jamali, M. A.; Yeh, C.; Yuen, L.; Griller, D. The DNA double-stranded break repair protein endo-exonuclease as a therapeutic target for cancer. Mol Cancer Ther 2004, 3, 911-9.
2. Pharma, O. Inhibitors of Endo-Exonuclease activity for treating cancer. 2001.
3. Pharma, O. Pentamidine Combinations for Treating Cancer. 2010.
4. Clement, B. Reduction of N-hydroxylated compounds: amidoximes (N-hydroxyamidines) as pro-drugs of amidines. Drug Metab Rev 2002, 34, 565-79.
5. Clement, B.; Schmitt, S.; Zimmermann, M. Enzymatic reduction of benzamidoxime to benzamidine. Arch Pharm (Weinheim) 1988, 321, 955-6.
6. Clement, B.; Immel, M.; Terlinden, R.; Wingen, F. J. Reduction of amidoxime derivatives to pentamidine in vivo. Arch Pharm (Weinheim) 1992, 325, 61-2.
7. Havemeyer, A.; Bittner, F.; Wollers, S.; Mendel, R.; Kunze, T.; Clement, B. Identification of the missing component in the mitochondrial benzamidoxime prodrug-converting system as a novel molybdenum enzyme. J Biol Chem 2006, 281, 34796-802.
8. Gruenewald, S.; Wahl, B.; Bittner, F.; Hungeling, H.; Kanzow, S.; Kotthaus, J.; Schwering, U.; Mendel, R. R.; Clement, B. The fourth molybdenum containing enzyme mARC: cloning and involvement in the activation of N-hydroxylated prodrugs. J Med Chem 2008, 51, 8173-7.
9. Clement, B.; Burenheide, A.; Rieckert, W.; Schwarz, J. Diacetyldiamidoximeester of pentamidine, a prodrug for treatment of protozoal diseases: synthesis, in vitro and in vivo biotransformation. ChemMedChem 2006, 1, 1260-7.
10. Clement, B. R., C. Improvement of the bioavailability of active substances having an amidine function in medicaments. 2008.
11. Clement, B. R., C.; Hungeling, H. Use of amidoxime carboxylic acid esters and N-hydroxyguanidine carboxylic acid esters for producing prodrugs. 2009.
12. Reeh, C.; Wundt, J.; Clement, B. N,N'-dihydroxyamidines: a new prodrug principle to improve the oral bioavailability of amidines. J Med Chem 2007, 50, 6730-4.
13. Arafa, R. K.; Brun, R.; Wenzler, T.; Tanious, F. A.; Wilson, W. D.; Stephens, C. E.; Boykin, D. W. Synthesis, DNA affinity, and antiprotozoal activity of fused ring dicationic compounds and their prodrugs. J Med Chem 2005, 48, 5480-8.
14. Brendle, J. J.; Outlaw, A.; Kumar, A.; Boykin, D. W.; Patrick, D. A.; Tidwell, R. R.; Werbovetz, K. A. Antileishmanial activities of several classes of aromatic dications. Antimicrob Agents Chemother 2002, 46, 797-807.
15. Donkor, I. O.; Huang, T. L.; Tao, B.; Rattendi, D.; Lane, S.; Vargas, M.; Goldberg, B.; Bacchi, C. Trypanocidal activity of conformationally restricted pentamidine congeners. J Med Chem 2003, 46, 1041-8.
16. Ismail, M. A.; Brun, R.; Wenzler, T.; Tanious, F. A.; Wilson, W. D.; Boykin, D. W. Dicationic biphenyl benzimidazole derivatives as antiprotozoal agents. Bioorg Med Chem 2004, 12, 5405-13.

## Patentansprüche

1. Verbindung der Formel 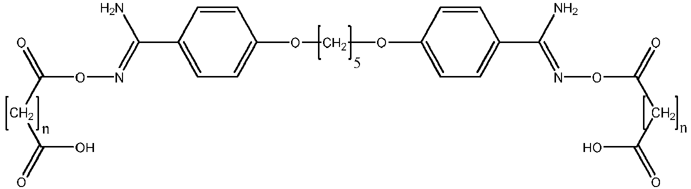 in welcher n für 2 steht.

2. Salze, Solvate und Solvate der Salze der Verbindungen gemäß Anspruch 1.

3. Verbindung nach einem der Ansprüche 1 bis 2 zur Verwendung bei der Behandlung und/oder Prophylaxe von Krankheiten.

4. Verbindung nach einem der Ansprüche 1 bis 2 zur Verwendung bei der Behandlung und/oder Prophylaxe von onkologischen Erkrankungen und Tumorerkrankungen jeglicher Pathogenese.

5. Verbindung nach einem der Ansprüche 1 bis 2 zur Verwendung bei der Behandlung und/oder Prophylaxe der Leishmaniose, Trypanosomiasis und/oder der Pneumocystis carinii Pneumonie (PcP).

6. Verbindung nach einem der Ansprüche 1 bis 2 zur Verwendung bei der Behandlung und/oder Prophylaxe der Malaria.

7. Arzneimittel umfassend mindestens eine Verbindung nach einem der Ansprüche 1 bis 2 gegebenenfalls in Kombination mit einem oder mehreren inerten, nicht-toxischen, pharmazeutisch geeigneten Hilfsstoffen.

8. Arzneimittel umfassend mindestens eine Verbindung nach einem der Ansprüche 1 bis 2 in Kombination mit einem oder mehreren weiteren Wirkstoffen.

9. Arzneimittel umfassend mindestens eine Verbindung nach einem der Ansprüche 1 bis 2 zur oralen oder parenteralen Anwendung.

10. Arzneimittel umfassend mindestens eine Verbindung nach einem der Ansprüche 1 bis 2 zur Behandlung und/oder Prophylaxe von Tumorerkrankungen.

11. Arzneimittel nach einem der Ansprüche 7 bis 10, **dadurch gekennzeichnet, dass** das Arzneimittel magensaftresistent formuliert ist.

12. Verfahren zur Herstellung einer Verbindung gemäß einem der Ansprüche 1 bis 2, **dadurch gekennzeichnet, dass** das Amidoxim der Formel (A) 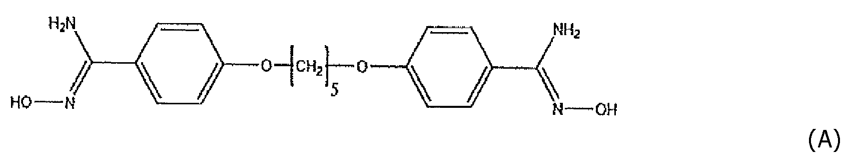 durch Reaktion mit einem Dicarbonsäureanhydrid der Formel (B) 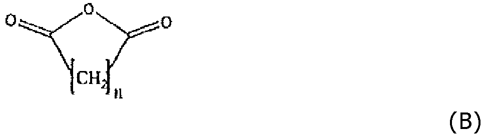 in welcher n für 2 steht
in eine Verbindung der Formel (C) 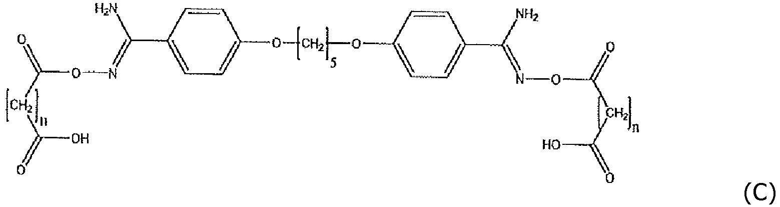 überführt wird.

## Claims

1. A compound of the formula 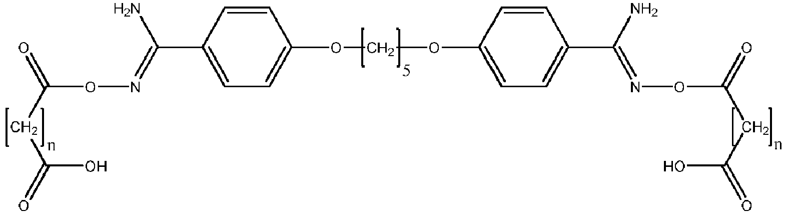 in which n represents 2.

2. Salts, solvates and solvates of the salts of the compounds according to claim 1.

3. The compound according to any one of claims 1 to 2 for use in the treatment and/or prophylaxis of diseases.

4. The compound according to any one of claims 1 to 2 for use in the treatment and/or prophylaxis of oncological diseases and tumorous diseases of any pathogenesis.

5. The compound according to any one of claims 1 to 2 for use in the treatment and/or prophylaxis of leishmaniasis, trypanosomiasis and/or pneumocystis carinii pneumonia (PcP).

6. The compound according to any one of claims 1 to 2 for use in the treatment and/or prophylaxis of malaria.

7. A drug comprising at least one compound according to any one of claims 1 to 2, if appropriate in combination with one or more inert, non-toxic, pharmaceutically suited excipients.

8. The drug comprising at least one compound according to any one of claims 1 to 2 in combination with one or more further active agents.

9. The drug comprising at least one compound according to any one of claims 1 to 2 for oral or parenteral administration.

10. The drug according to any one of claims 1 to 2 for the treatment and/or prophylaxis of tumorous diseases.

11. The drug according to any one of claims 1 to 10, **characterized in that** the drug is of enteric formulation.

12. A method for preparing a compound according to any one of claims 1 to 2, **characterized in that** the amidoxime of the formula (A) 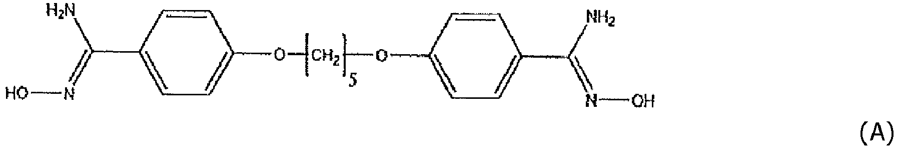 is converted by reacting with dicarboxylic acid anhydride of the formula (B) 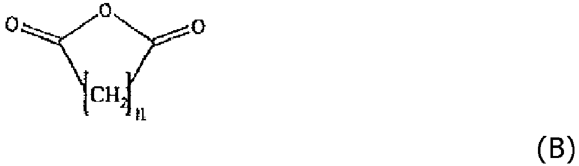 in which n represents 2,
into a compound of the formula (C)

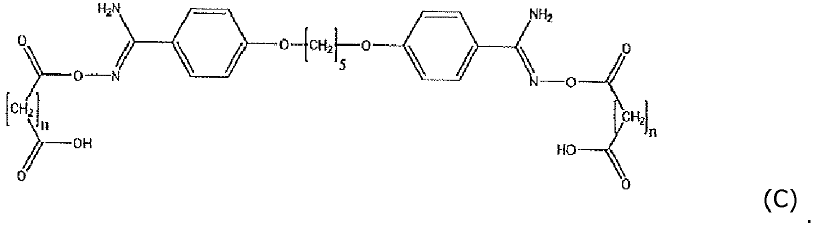

## Revendications

1. Liaison de la formule 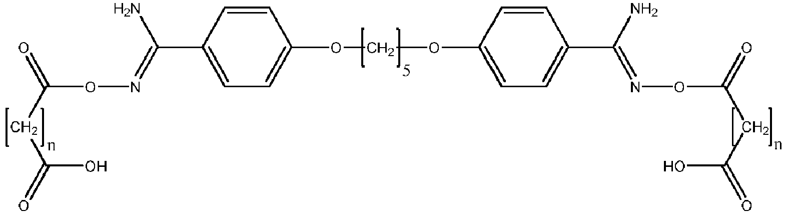 dans laquelle n représente 2.

2. Sels, solvates et solvates des sels des liaisons selon la revendication 1.

3. Liaison selon l'une des revendications 1 à 2 destinée à être utilisée dans le traitement et/ou la prophylaxie de maladies.

4. Liaison selon l'une des revendications 1 à 2 destinée à être utilisée dans le traitement et/ou la prophylaxie d'affections oncologiques et d'affections tumorales de toute pathogenèse.

5. Liaison selon l'une des revendications 1 à 2 destinée à être utilisée dans le traitement et/ou la prophylaxie de la leishmaniose, de la trypanosomiase et/ou de Pneumocystis carinii Pneumonie (PcP).

6. Liaison selon l'une des revendications 1 à 2 destinée à être utilisée dans le traitement et/ou la prophylaxie de la malaria.

7. Médicament comprenant au moins une liaison selon l'une des revendications 1 à 2 éventuellement en combinaison avec une ou plusieurs substances auxiliaires inertes, non toxiques, appropriées pharmaceutiquement.

8. Médicament comprenant au moins une liaison selon l'une des revendications 1 à 2 en combinaison avec un ou plusieurs agents actifs supplémentaires.

9. Médicament comprenant au moins une liaison selon l'une des revendications 1 à 2 pour application orale ou parentérale.

10. Médicament comprenant au moins une liaison selon l'une des revendications 1 à 2 pour le traitement et/ou la prophylaxie d'affections tumorales.

11. Médicament selon l'une des revendications 7 à 10, **caractérisé en ce que** le médicament est formulé de façon résistante au suc gastrique.

12. Procédé de fabrication d'une liaison selon l'une des revendications 1 à 2, **caractérisé en ce que** l'amidoxime de la formule (A) 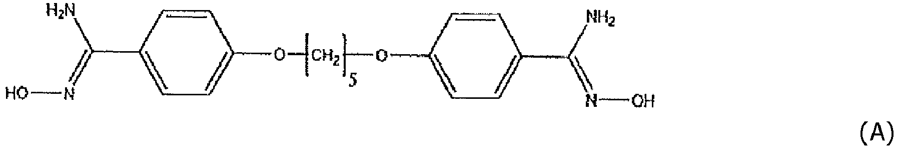 est transformé par réaction avec un anhybride d'acide dicarbonique de la formule (B) 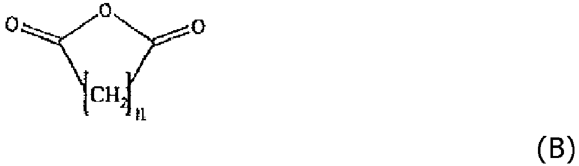 dans lequel n représente 2 en une liaison de la formule (C)
